# FASCICULE DE BREVET EUROPEEN

(11) **EP 4 076 747 B1**
(45) Date de publication et mention de la délivrance du brevet: **07.02.2024**
(21) Numéro de dépôt: 20851206.1
(22) Date de dépôt: 18.12.2020
(51) Int. Cl.: B01L 1/02, A61L 2/26, G21F 7/005, B25J 21/02

(54) **CONTENEUR ETANCHE COMPRENANT UN DISPOSITIF PERMETTANT UN RACCORDEMENT AMOVIBLE A UNE ENCEINTE**
VERSIEGELTER BEHÄLTER MIT EINER VORRICHTUNG ZUR LÖSBAREN VERBINDUNG MIT EINEM GEHÄUSE
SEALED CONTAINER COMPRISING A DEVICE ALLOWING REMOVABLE CONNECTION TO AN ENCLOSURE

(30) Priorité: 20.12.2019 FR 1915376
(43) Date de publication de la demande: 26.10.2022
(73) Titulaire: ABC Transfer, 37000 Tours (FR)
(72) Inventeur: FELIX, Julien, 41100 VENDOME (FR); SCHNEIDER, Jean-Luc, 41100 SAINT FIRMIN DES PRES (FR); GIRARD, Thierry, 75116 PARIS (FR)
(74) Mandataire: IP Trust
(86) Numéro de dépôt international: PCT/FR2020/052548
(87) Numéro de publication internationale: WO 2021/123676

(56) Documents cités:
- WO-A1-2014/172665
- FR-A1- 2 951 516
- US-A1- 2003 155 846

## Description

### DOMAINE TECHNIQUE DE L'INVENTION

L'invention concerne le domaine des conteneurs destinés à être raccordés de manière étanche à des enceintes stériles afin de permettre la mise en communication de leurs volumes respectifs sans contact avec ledit milieu extérieur.

L'invention concerne plus particulièrement un conteneur étanche comprenant un corps de conteneur présentant une ouverture de passage délimitée par une bride et obturée par une porte amovible montée sur la bride, un joint d'étanchéité annulaire situé entre la bride et la porte, la bride, la porte et le joint d'étanchéité formant un dispositif de connexion étanche apte à être raccordé à un dispositif de connexion complémentaire d'une enceinte stérile par des moyens de raccordement à baïonnette en vue de permettre un communication stérile entre le conteneur étanche et l'enceinte stérile.

Le conteneur étanche selon l'invention est destiné notamment, mais non exclusivement au transfert de produits dangereux comme certains produits pharmaceutiques, biotechnologiques, biologiques, chimiques ou radioactifs, du transfert de composants tels que bouchons, flacons, pistons, seringues, etc., au transfert de dispositifs de contrôle environnementaux tels que des supports de milieu de culture, des compteurs particulaires, etc., du transfert de systèmes de nettoyage, du transfert de liquides, de poudres, d'outils, du transfert de déchets vers l'extérieur de l'enceinte et/ou du transfert de tout élément nécessaire à la production ou à la maintenance de la ligne de production.

### ETAT DE LA TECHNIQUE

De manière connu, le raccordement d'un conteneur étanche à une enceinte stérile pour le transfert de produits, sans rupture de la connexion et de l'étanchéité, est réalisé à l'aide de deux dispositifs de connexion, l'un équipant le conteneur, l'autre équipant l'enceinte. Chaque dispositif de connexion comporte une bride délimitant respectivement l'ouverture de passage dans le volume intérieur du conteneur ou de l'enceinte, chacune des ouvertures de passage étant obturées par une porte. La bride et la porte du conteneur sont aptes à être raccordées respectivement à la bride et à la porte de l'enceinte par une liaison à baïonnettes sous l'action d'un mouvement de rotation de la bride et porte associée du conteneur par rapport à la bride et porte de l'enceinte sur lesquelles elles sont accolées. Ainsi, les brides et les portes respectivement du conteneur et de l'enceinte sont pourvues respectivement d'encoches et d'oreilles internes ou externes. Lors du mouvement de rotation, la porte du conteneur est désolidarisée de la bride associée. WO 2014/172665 A1 divulgue un conteneur selon l'état de la technique.

Ces dispositifs de connexion donnent dans l'ensemble satisfaction. Cependant, les frottements générés lors du mouvement de rotation effectué pour procéder au verrouillage de la porte sur la bride présentent l'inconvénient de produire des particules susceptibles d'induire une contamination au sein des volumes clos mais également de produire une rupture d'étanchéité du raccordement. Par ailleurs, les oreilles internes de la bride assurant l'accroche de la porte s'étendent, une fois la porte désengagée de ces oreilles, dans l'ouverture de passage, de sorte que le transfert des composants peut être parfois gêné par ces oreilles.

L'invention vise à remédier à ces problèmes en proposant un conteneur étanche limitant l'émission de particules susceptibles de générer une contamination des volumes clos lors le de l'assemblage de la porte sur la bride associée du conteneur.

L'invention a également pour objet de proposer un conteneur étanche offrant une ouverture de passage optimisée ainsi qu'une étanchéité stable et durable entre le conteneur et l'enceinte à laquelle il est raccordé.

### OBJET DE L'INVENTION

A cet effet, l'invention propose un conteneur étanche comprenant un corps de conteneur présentant une ouverture de passage délimitée par une bride et obturée par une porte amovible montée sur la bride, un joint d'étanchéité annulaire situé entre la bride et la porte, la bride, la porte et le joint d'étanchéité formant un dispositif de connexion étanche apte à être raccordé à un dispositif de connexion complémentaire d'une enceinte stérile par des moyens de raccordement à baïonnette en vue de permettre une communication stérile entre le conteneur étanche et l'enceinte stérile, le conteneur étant remarquable en ce que le conteneur comporte un bourrelet annulaire d'accroche de la porte sur la bride s'enfichant dans une encoche de la porte prévue à cet effet.

La porte est ainsi assemblée à la bride du conteneur par simple clipsage et de la même manière, désassemblée par déclipsage.

En s'affranchissant ainsi du mouvement de rotation imposé par la liaison à baïonnettes mise en oeuvre dans les conteneurs de l'art antérieur, la production de particules génératrices de contamination est évitée. Le bourrelet d'accroche présente également l'avantage d'offrir un encombrement moins important que les oreilles des brides. Cela permet ainsi d'offrir un diamètre de passage augmenté et ainsi de faciliter le passage des composants et de réduire les risques d'endommagement de ces derniers lors de leur transfert.

Selon un mode de réalisation particulier, le bourrelet annulaire d'accroche est ménagé sur le pourtour périphérique intérieur de la bride.

Selon un autre mode de réalisation, le bourrelet annulaire d'accroche est déporté sur le joint d'étanchéité. Ainsi, ledit le bourrelet est ménagé sur le pourtour périphérique intérieur dudit joint d'étanchéité. L'accroche de la porte sur la bride est ainsi réalisée par le biais du joint d'étanchéité.

Avantageusement, la bride est constituée de deux corps de brides coaxiaux distincts, l'un des corps de brides définissant un corps de bride externe arrangé pour permettre une connexion du corps de conteneur à l'enceinte complémentaire, l'autre corps de bride définissant un corps de bride interne, lesdits corps de brides étant arrangés l'un par rapport à l'autre pour définir entre eux un espace interstitiel annulaire dans lequel le joint d'étanchéité est logé.

Avantageusement, le bourrelet annulaire d'accroche est ménagé sur le pourtour périphérique intérieur du corps de bride interne. Dans le cas cependant où le bourrelet annulaire d'accroche est déporté sur le joint d'étanchéité, ce dernier et le corps de bride interne sont avantageusement arrangés pour que le bourrelet d'accroche repose sur l'extrémité avant du corps de bride interne.

Avantageusement, le conteneur comporte des moyens anti-rotatifs de la porte (7) sur la bride. Selon une variante préférée, le bourrelet présente un profil aménagé pour assurer l'anti- rotation de la porte sur la bride.

Avantageusement, le joint d'étanchéité comporte une portion de face crantée destinée à être au contact de la porte.

Avantageusement, le joint d'étanchéité comporte des moyens d'association réciproque avec la bride.

Avantageusement, le joint d'étanchéité présente au moins une face de contact avec l'un au moins des corps de bride pourvue d'un crantage coopérant avec un crantage complémentaire ménagé sur la face dudit corps de bride en vis-à-vis de laquelle elle est placée, le crantage de la face de contact du joint et le crantage complémentaire formant les moyens d'association réciproque.

Avantageusement, le corps de bride externe comporte un rebord périphérique interne en butée duquel le joint d'étanchéité est positionné.

Avantageusement, le corps de conteneur est formé par un film souple fixé sur la bride, le film souple étant maintenu en prise entre le corps de bride externe et le corps de bride interne.

Il peut être prévu également que le corps de bride externe comporte une jupe inférieure comprenant des fentes longitudinales s'étendant depuis la bordure d'extrémité inférieure du corps de bride externe et équiréparties sur la périphérie dudit corps de bride de sorte à former des languettes élastiques aptes à exercer un effort de serrage sur le corps de bride interne. Il peut être prévu également que le conteneur comporte une bague de serrage du corps de bride externe sur le corps de bride interne arrangé pour enserrer les parties du corps de bride externe et du corps de bride interne entre lesquelles le film souple est interposé. Avantageusement, le conteneur comporte un joint annulaire additionnel logé dans une gorge périphérique ménagée sur la face interne du corps de bride externe, le film souple s'étendant entre le joint annulaire additionnel et le corps de bride interne. Ces caractéristiques, prises seules ou en combinaison entre elles, permettent d'améliorer la tenue mécanique du conteneur et d'assurer une étanchéité optimale. Elles permettent également de réaliser de manière simple et rapide un conteneur, sans risque de détérioration du film et de contamination du contenant de ce dernier.

Avantageusement, tout ou partie du conteneur est réalisé en un matériau bactériostatique.

### BREVE DESCRIPTION DES FIGURES

D'autres caractéristiques et avantages de l'invention ressortiront de la description détaillée de l'invention qui va suivre en référence aux figures annexées et dans lesquelles :
- La figure 1 représente une vue schématique de face d'un conteneur selon l'invention ;
- La figure 2 représente une vue en coupe longitudinale partielle du conteneur de la figure 1 ;
- La figure 3 représente une vue de détail du conteneur de la figure 2 ;
- La figure 4 représente une vue partielle du corps de bride externe du dispositif de connexion du conteneur de la figure 2 ;
- La figure 5 représente le conteneur de la figure 2 pourvu d'un système de chute pour le transfert de composant ;
- La figure 6 représente une vue de détail du conteneur de la figure 5 ;
- La figure 7 représente une vue en coupe longitudinale partielle d'un autre exemple de réalisation d'un conteneur selon l'invention.

Pour plus de clarté, les éléments identiques ou similaires des différents modes de réalisation sont repérés par des signes de référence identiques sur l'ensemble des figures.

### DESCRIPTION DETAILLEE DE L'INVENTION

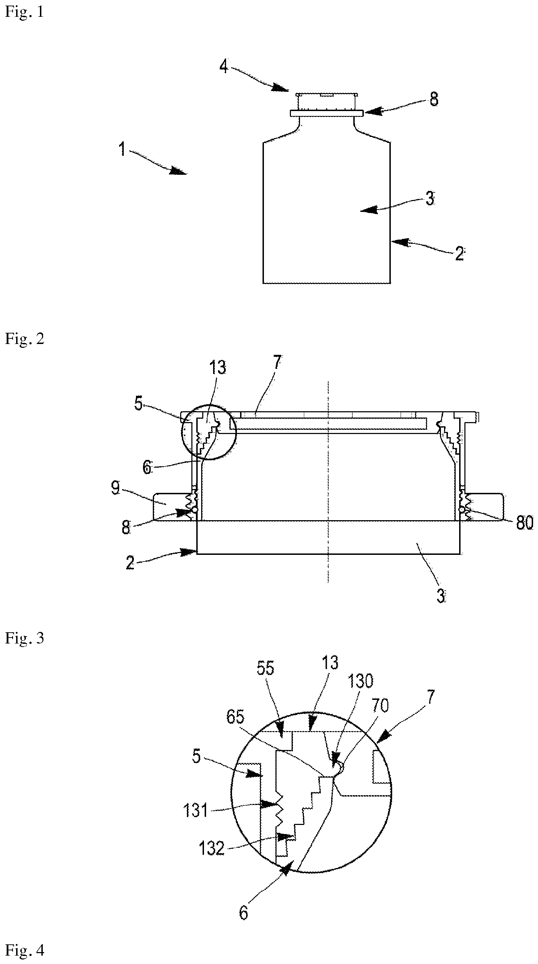

En relation avec les figures 1 à 6, il est décrit un conteneur 1 selon un premier exemple de réalisation de l'invention. Le conteneur 1 illustré comprend un film souple 2 arrangé pour définir un volume intérieur 3 et une ouverture de passage permettant de mettre en communication le volume intérieur 3 avec l'extérieur du film.

Le conteneur comporte également un dispositif de connexion étanche lequel est arrangé pour permettre la connexion du film souple 2 à un dispositif de connexion complémentaire d'une enceinte stérile. La connexion est réalisée de manière à assurer une communication stérile entre le volume intérieur 3 dudit conteneur 1 et celui de l'enceinte. Le dispositif de connexion comprend une bride sur laquelle le film souple est fixé, ladite bride délimitant l'ouverture de passage du film souple. Il comprend en outre une porte 7 amovible fixée sur la bride de façon à obturer l'ouverture de passage.

Selon l'invention, la bride est constituée de deux pièces distinctes l'une de l'autre, une première pièce formant un corps de bride dit externe et une deuxième pièce formant un corps de bride dit interne, entre lesquels le film souple s'étend.

Le corps de bride interne 6 comporte une zone crantée adjacente au renfoncement d'appui arrangée pour coopérer avec un crantage complémentaire ménagé sur la face interne du corps de bride externe 5.

Le corps de bride externe 5 comporte, selon une configuration avantageuse illustrée sur la figure 4, une jupe inférieure 10 comprenant des fentes longitudinales 11 s'étendant depuis la bordure d'extrémité inférieure dudit corps de bride. Ces fentes longitudinales 11 sont avantageusement équiréparties sur la périphérie dudit corps de bride. Elles délimitent ainsi des languettes élastiques 12 aptes à exercer un effort de serrage homogène sur l'ensemble de la périphérie extérieure du corps de bride interne 6 lorsque le corps de bride externe 5 est monté sur le corps de bride interne 6. Les languettes élastiques 12 comportent des moyens d'association réciproque avec respectivement des moyens de serrage, décrite ci-après, et le corps de bride interne 6. Dans l'exemple illustré, les moyens d'association réciproque avec la bague de serrage consistent en un filetage et un crantage. Les moyens de serrage sont arrangés pour enserrer les parties du corps de bride externe 5 et du corps de bride interne 6 entre lesquelles le film souple 2 est interposé. Dans l'exemple illustré, les moyens de serrage se présentent sous la forme d'une bague de serrage externe comprenant un taraudage de forme complémentaire à un filetage ménagé sur la face externe du corps de bride externe 5.

Le conteneur 1 comporte en outre un premier joint annulaire 8 logé dans une gorge périphérique ménagée sur la face interne du corps de bride externe 5 et la face externe du corps de bride interne 6. Comme montré sur la figure 3, le film souple 2 est maintenu en prise entre le joint annulaire 8 et le corps de bride interne 6. Afin d'améliorer la retenue du film souple 2 entre les deux corps de brides constitutifs de la bride, le corps de bride interne 6 comporte une face externe pourvue d'un renfoncement d'appui situé en vis-à-vis de la gorge périphérique du corps de bride externe 5. Ainsi, lors du serrage du corps de bride externe 5 sur le corps de bride interne 6, le joint annulaire 8 écrase le film souple 2 au niveau du renfoncement d'appui, assurant une mise en prise améliorée du film souple 2.

Le conteneur 1 comporte un deuxième joint annulaire 13 lequel est porté par la bride. Il définit un joint d'étanchéité porte/bride. Lorsque la porte est en position d'obturation de l'ouverture de passage, le joint annulaire 13 est logé dans l'espace délimité par le corps de bride externe 5, le corps de bride interne 6 et la porte 7. Avantageusement, le joint annulaire 13 présente une section de forme conjuguée à l'espace dans lequel il est logé. Dans le mode de réalisation illustré, le joint annulaire 13 présente une section sensiblement triangulaire.

Comme illustré sur les figures 2 et 3, et selon l'invention, la porte 7 est maintenue sur la bride par l'intermédiaire du joint d'étanchéité 13. A cet effet, celui-ci présente, sur son pourtour périphérique intérieur, un bourrelet d'accroche 130 annulaire apte à s'enficher dans une encoche 70 ménagée sur le bord périphérique extérieur de la porte 7.

Afin d'éviter un affaissement du bourrelet d'accroche 130, le joint d'étanchéité 13 est arrangé avec le corps de bride interne 6 de manière à ce que le bourrelet d'accroche 130 repose sur l'extrémité avant 65 du corps de bride interne 6.

Afin d'assurer le maintien du joint d'étanchéité 130 entre les corps de bride externe et interne 5, 6, celui-ci comporte des moyens d'association réciproque avec le corps de bride externe 5 d'une part et le corps de bride interne 6 d'autre part. Dans le mode de réalisation illustré, les moyens d'association réciproque se présentent sous la forme de zones crantées. Ainsi, les faces du joint d'étanchéité en contact avec le corps de bride externe 5 et le corps de bride externe 6 comportent un crantage 131, 132 coopérant avec un crantage complémentaire ménagé sur les faces desdits corps de brides en vis-à-vis desquelles elles sont placées. Dans l'exemple illustré, la face de contact 132 avec le corps de bride interne présente en particulier une forme en escalier afin d'assurer un maintien renforcé du joint d'étanchéité 13 entre les corps de bride externe et interne 5, 6. Avantageusement, le corps de bride externe 5 est pourvu d'un rebord périphérique 55 interne en butée duquel le joint d'étanchéité 13 est positionné, assurant ainsi une retenue axiale dudit joint, notamment lors du retrait de la porte 7. Le joint d'étanchéité 13 et son assemblage avec la bride selon l'arrangement décrit précédemment permet ainsi de supprimer tout risque de déformation, de torsion et de déplacement du joint d'étanchéité 13 lors de la réalisation du dispositif de connexion ainsi que lors de l'utilisation du conteneur.

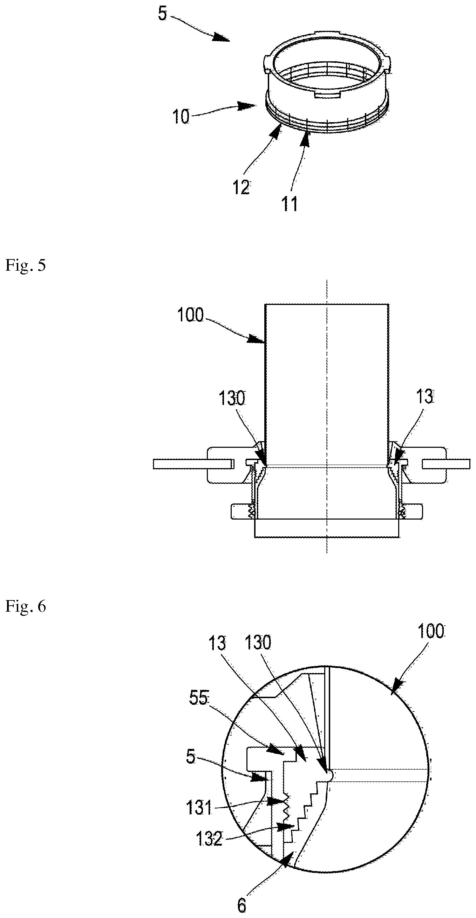

Avantageusement, le bourrelet d'accroche 130 est arrangé pour permettre l'accostage d'un système de chute 100 pour le transfert de composant. Comme illustré sur les figures 5 et 6, ce dernier est monté en appui contre le bourrelet d'accroche 130.

Du fait de l'arrangement des éléments le composant, le dispositif de connexion est assemblé par simple clipsage des éléments. Pour réaliser le conteneur 1, la partie du film souple, située du côté de l'ouverture de passage, sera disposée de façon à être placée entre le corps de bride externe 5 et le corps de bride interne 6. Le serrage sera effectif par serrage de la bague 9 sur le corps de bride externe 6, entrainant le resserrement des languettes 12 contre le corps de bride interne 6. Le film souple 2 pourra être désassemblé en dévissant la bague de serrage 9 et en déclipsant le corps de bride externe 6 du corps de bride interne 7.

Le film souple 2 et le dispositif de connexion sont avantageusement réalisés en un matériau bactériostatique. Les joints d'étanchéité 8 et 13 sont réalisés en un matériau silicone ou autres matériaux minimisant les phénomènes de rémanence. Il peut être prévu également que les joints d'étanchéité soient réalisés en un matériau bactériostatique.

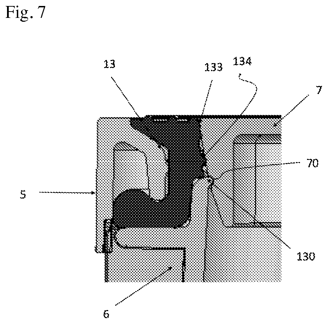

La figure 7 illustre un autre exemple de réalisation de montage de la porte 7 sur la bride. Dans cet exemple de réalisation, la bride comprend également un corps de bride externe 5 et un corps de bride interne 6. Le bourrelet annulaire d'accroche 130 est en revanche porté par le corps de bride interne. Avantageusement, la portion de face 133 du joint d'étanchéité 13 destinée à être au contact de la porte 7 présente un crantage 134. Le terme crantage s'entend par toute forme rendant la face du joint d'étanchéité non plane de sorte à favoriser les frottements. Le terme « crantage » comprend ainsi par extension des stries, un crénelage, un bosselage, etc. Un tel arrangement du joint d'étanchéité avec le bourrelet annulaire d'accroche 130 porté par la bride 7 présente l'avantage d'améliorer le maintien de la porte 7 sur la bride et conjointement de renforcer l'étanchéité entre la porte 7 et le corps de bride interne 6.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Conteneur (1) étanche comprenant :
- un corps de conteneur (2) présentant une ouverture de passage (8) délimitée par une bride et obturée par une porte (7) amovible montée sur la bride,
- un joint d'étanchéité (13) annulaire situé entre la bride et la porte (7),
- la bride, la porte (7) et le joint d'étanchéité formant un dispositif de connexion étanche apte à être raccordé à un dispositif de connexion complémentaire d'une enceinte stérile par des moyens de raccordement à baïonnette en vue de permettre une communication stérile entre le conteneur étanche et l'enceinte stérile,
**caractérisé en ce que** le conteneur (1) comporte un bourrelet annulaire d'accroche (130) de la porte (7) sur la bride s'enfichant dans une encoche (70) de ladite porte (7) prévue à cet effet, de sorte à permettre un assemblage de la porte (7) sur la bride par clipsage, et inversement.

2. Conteneur étanche (1) selon la revendication 1, **caractérisé en ce que** le bourrelet annulaire d'accroche (130) est ménagé sur le pourtour périphérique intérieur de la bride.

3. Conteneur étanche (1) selon la revendication 1, **caractérisé en ce que** le bourrelet annulaire d'accroche (130) est ménagé sur le pourtour périphérique intérieur du joint d'étanchéité (13).

4. Conteneur étanche (1) selon l'une quelconque des revendications 1 à 3, **caractérisé en ce que** la bride est constituée de deux corps de brides coaxiaux distincts, l'un des corps de brides définissant un corps de bride externe (5) arrangé pour permettre une connexion du corps de conteneur (2) à l'enceinte complémentaire, l'autre corps de bride définissant un corps de bride interne (6), lesdits corps de brides étant arrangés l'un par rapport à l'autre pour définir entre eux un espace interstitiel annulaire dans lequel le joint d'étanchéité (13) est logé.

5. Conteneur étanche (1) selon la revendication 4 lorsqu'elle dépend de la revendication 2, **caractérisé en ce que** le bourrelet annulaire d'accroche (130) est ménagé sur le pourtour périphérique intérieur du corps de bride interne (6).

6. Conteneur étanche (1) selon la revendication 4 lorsqu'elle dépend de la revendication 3, **caractérisé en ce que** le joint d'étanchéité (13) et le corps de bride interne (6) sont arrangés pour que le bourrelet d'accroche (13) repose sur l'extrémité avant (65) du corps de bride interne.

7. Conteneur étanche (1) selon l'une quelconque des revendications précédentes, **caractérisé en ce qu'**il comporte des moyens anti-rotatifs de la porte (7) sur la bride.

8. Conteneur étanche (1) selon l'une quelconque des revendications précédentes, **caractérisé en ce que** le joint d'étanchéité (13) comporte une portion de face (133) crantée destinée à être au contact de la porte (7).

9. Conteneur étanche (1) selon l'une quelconque des revendications précédentes, **caractérisé en ce que** le joint d'étanchéité (13) comporte des moyens d'association réciproque avec la bride.

10. Conteneur étanche (1) selon la revendication 4 et l'une quelconque des revendications 5 à 9 lorsqu'elles dépendent de la revendication 4, **caractérisé en ce que** le joint d'étanchéité (13) présente au moins une face de contact avec l'un au moins des corps de bride pourvue d'un crantage coopérant avec un crantage complémentaire ménagé sur la face dudit corps de bride en vis-à-vis de laquelle elle est placée, le crantage de la face de contact du joint et le crantage complémentaire formant les moyens d'association réciproque.

11. Conteneur étanche (1) selon la revendication 4 ou l'une quelconque des revendications 5 à 10 lorsqu'elles dépendent de la revendication 4, **caractérisé en ce que** le corps de bride externe (5) comporte un rebord périphérique interne (55) en butée duquel le joint d'étanchéité (13) est positionné.

12. Conteneur (1) étanche selon la revendication 4 ou l'une quelconque des revendications 5 à 11 lorsqu'elles dépendent de la revendication 4, **caractérisé en ce que** le corps de bride externe (5) comporte une jupe inférieure (10) comprenant des fentes longitudinales (11) s'étendant depuis la bordure d'extrémité inférieure du corps de bride externe (5) et équiréparties sur la périphérie dudit corps de bride de sorte à former des languettes élastiques (12) aptes à exercer un effort de serrage sur le corps de bride interne (6).

13. Conteneur (1) étanche selon la revendication 4 ou l'une quelconque des revendications 5 à 12 lorsqu'elles dépendent de la revendication 4, **caractérisé en ce que** le corps de conteneur est formé par un film souple (2) fixé sur la bride, le film souple (2) étant maintenu en prise entre le corps de bride externe (5) et le corps de bride interne (6).

14. Conteneur (1) étanche selon la revendication 13, **caractérisé en ce qu'**il comporte une bague de serrage (20) du corps de bride externe (5) sur le corps de bride interne (6) arrangé pour enserrer les parties du corps de bride externe (5) et du corps de bride interne (6) entre lesquelles le film souple est interposé.

15. Conteneur (1) étanche selon la revendication 13 ou la revendication 14, **caractérisé en ce qu'**il comporte un joint annulaire (8) additionnel logé dans une gorge périphérique ménagée sur la face interne du corps de bride externe (5), le film souple (2) s'étendant entre le joint annulaire (8) additionnel et le corps de bride interne (6).

16. Conteneur (1) étanche selon l'une quelconque des revendications précédentes, **caractérisé en ce que** tout ou partie du conteneur est réalisé en un matériau bactériostatique.

## Patentansprüche

1. Dichter Behälter (1), umfassend:
- einen Behälterkörper (2), der eine Durchgangsöffnung (8) vorweist, die durch einen Flansch begrenzt wird und durch eine abnehmbare Tür (7) verschlossen wird, die an dem Flansch montiert ist,
- eine ringförmige Dichtung (13), die sich zwischen dem Flansch und der Tür (7) befindet,
- wobei der Flansch, die Tür (7) und die Dichtung eine dichte Verbindungsvorrichtung ausbilden, die geeignet ist, um mit einer komplementären Verbindungsvorrichtung eines sterilen abgeschlossenen Raums durch Bajonettanschlussmittel angeschlossen zu werden, um eine sterile Kommunikation zwischen dem dichten Behälter und dem sterilen abgeschlossenen Raum zu ermöglichen,
**dadurch gekennzeichnet, dass** der Behälter (1) einen ringförmigen Einhakwulst (130) der Tür (7) an dem Flansch aufweist, der sich in eine zu diesem Zweck vorgesehene Kerbe (70) der Tür (7) einsteckt, so dass ein Zusammenbau der Tür (7) an dem Flansch durch Einklipsen und umgekehrt möglich ist.

2. Dichter Behälter (1) nach Anspruch 1, **dadurch gekennzeichnet, dass** der ringförmige Einhakwulst (130) an der inneren Umfangsumrandung des Flansches eingerichtet ist.

3. Dichter Behälter (1) nach Anspruch 1, **dadurch gekennzeichnet, dass** der ringförmige Einhakwulst (130) an der inneren Umfangsumrandung der Dichtung (13) eingerichtet ist.

4. Dichter Behälter (1) nach einem der Ansprüche 1 bis 3,
**dadurch gekennzeichnet, dass** der Flansch aus zwei verschiedenen koaxialen Flanschkörpern besteht, wobei einer der Flanschkörper einen äußeren Flanschkörper (5) definiert, der angeordnet ist, um eine Verbindung des Behälterkörpers (2) mit dem komplementären abgeschlossenen Raum zu ermöglichen, wobei der andere Flanschkörper einen inneren Flanschkörper (6) definiert, wobei die Flanschkörper relativ zueinander angeordnet sind, um zwischen ihnen einen ringförmigen Zwischenraum zu definieren, in dem die Dichtung (13) untergebracht ist.

5. Dichter Behälter (1) nach Anspruch 4, wenn er von Anspruch 2 abhängt, **dadurch gekennzeichnet, dass** der ringförmige Einhakwulst (130) an der inneren Umfangsumrandung des inneren Flanschkörpers (6) eingerichtet ist.

6. Dichter Behälter (1) nach Anspruch 4, wenn er von Anspruch 3 abhängt, **dadurch gekennzeichnet, dass** die Dichtung (13) und der innere Flanschkörper (6) so angeordnet sind, dass der Einhakwulst (13) auf dem vorderen Ende (65) des inneren Flanschkörpers aufliegt.

7. Dichter Behälter (1) nach einem der vorstehenden Ansprüche, **dadurch gekennzeichnet, dass** er Drehsicherungsmittel der Tür (7) auf dem Flansch umfasst.

8. Dichter Behälter (1) nach einem der vorstehenden Ansprüche,
**dadurch gekennzeichnet, dass** die Dichtung (13) einen verzahnten Flächenabschnitt (133) aufweist, der dazu bestimmt ist, mit der Tür (7) in Kontakt zu sein.

9. Dichter Behälter (1) nach einem der vorstehenden Ansprüche, **dadurch gekennzeichnet, dass** die Dichtung (13) Mittel für eine gegenseitige Zuordnung mit dem Flansch aufweist.

10. Dichter Behälter (1) nach Anspruch 4 und einem der Ansprüche 5 bis 9, wenn sie von Anspruch 4 abhängen, **dadurch gekennzeichnet, dass** die Dichtung (13) mindestens eine Kontaktfläche mit mindestens einem der Flanschkörper vorweist, die mit einer Verzahnung versehen ist, die mit einer komplementären Verzahnung zusammenwirkt, die auf der Fläche des Flanschkörpers eingerichtet ist, derer sie gegenüber platziert ist, wobei die Verzahnung der Kontaktfläche der Dichtung und die komplementäre Verzahnung die Mittel für die gegenseitige Zuordnung ausbilden.

11. Dichter Behälter (1) nach Anspruch 4 oder einem der Ansprüche 5 bis 10, wenn sie von Anspruch 4 abhängen, **dadurch gekennzeichnet, dass** der äußere Flanschkörper (5) einen inneren Umfangsrand (55) aufweist, an dessen Anschlag die Dichtung (13) positioniert ist.

12. Dichter Behälter (1) nach Anspruch 4 oder einem der Ansprüche 5 bis 11, wenn sie von Anspruch 4 abhängen, **dadurch gekennzeichnet, dass** der äußere Flanschkörper (5) eine untere Schürze (10) aufweist, umfassend Längsschlitze (11), die sich von der unteren Endkante des äußeren Flanschkörpers (5) aus erstrecken und gleichmäßig über den Umfang des Flanschkörpers verteilt sind, so dass sie elastische Zungen (12) ausbilden, die geeignet sind, eine Klemmkraft auf den inneren Flanschkörper (6) auszuüben.

13. Dichter Behälter (1) nach Anspruch 4 oder einem der Ansprüche 5 bis 12, wenn sie von Anspruch 4 abhängen, **dadurch gekennzeichnet, dass** der Behälterkörper aus einer flexiblen Folie (2) ausgebildet ist, die an dem Flansch befestigt ist, wobei die flexible Folie (2) in Eingriff zwischen dem äußeren Flanschkörper (5) und dem inneren Flanschkörper (6) gehalten wird.

14. Dichter Behälter (1) nach Anspruch 13, **dadurch gekennzeichnet, dass** er einen Klemmring (20) des äußeren Flanschkörpers (5) auf dem inneren Flanschkörper (6) aufweist, der so angeordnet ist, dass er die Teile des äußeren Flanschkörpers (5) und des inneren Flanschkörpers (6), zwischen denen die flexible Folie liegt, umschließt.

15. Dichter Behälter (1) nach Anspruch 13 oder 14,
**dadurch gekennzeichnet, dass** er eine zusätzliche Ringdichtung (8) aufweist, die in einer Umfangsnut untergebracht ist, die an der Innenseite des äußeren Flanschkörpers (5) eingerichtet ist, wobei sich die flexible Folie (2) zwischen der zusätzlichen Ringdichtung (8) und dem inneren Flanschkörper (6) erstreckt.

16. Dichter Behälter (1) nach einem der vorstehenden Ansprüche, **dadurch gekennzeichnet, dass** der gesamte Behälter oder ein Teil davon aus einem bakteriostatischen Material hergestellt ist.

## Claims

1. Sealed container (1) comprising:
- a container body (2) having a through-hole (8) delimited by a flange and closed by a removable door (7) mounted on the flange,
- an annular seal (13) located between the flange and the door (7),
- the flange, the door (7) and the seal forming a sealed connection device capable of being connected to a complementary connection device of a sterile enclosure by means of bayonet connection means in order to allow sterile communication between the sealed container and the sterile enclosure,
**characterized in that** the container (1) comprises an annular attachment bead (130) for attaching the door (7) to the flange inserted into a notch (70) of said door (7) provided for this purpose, so as to allow an assembly of the door (7) on the flange by clipping, and vice versa.

2. Sealed container (1) according to claim 1, **characterized in that** the annular attachment bead (130) is provided on the inner peripheral circumference of the flange.

3. Sealed container (1) according to claim 1, **characterized in that** the annular attachment bead (130) is provided on the inner peripheral circumference of the seal (13).

4. Sealed container (1) according to one of claims 1 to 3, **characterized in that** the flange consists of two separate coaxial flange bodies, one of the flange bodies defining an external flange body (5) arranged to allow connection of the container body (2) to the complementary enclosure, the other flange body defining an internal flange body (6), said flange bodies being arranged relative to each other to define therebetween an annular interstitial space in which the seal (13) is housed.

5. Sealed container (1) according to claim 4 when it depends on claim 2, **characterized in that** the annular attachment bead (130) is provided on the inner peripheral circumference of the internal flange body (6).

6. Sealed container (1) according to claim 4 when it depends on claim 3, **characterized in that** the seal (13) and the internal flange body (6) are arranged so that the attachment bead (13) rests on the front end (65) of the internal flange body.

7. Sealed container (1) according to one of the preceding claims, **characterized in that** it comprises anti-rotation means preventing the rotation of the door (7) on the flange.

8. Sealed container (1) according to one of the preceding claims, **characterized in that** the seal (13) has a notched face portion (133) intended to be in contact with the door (7).

9. Sealed container (1) according to one of the preceding claims, **characterized in that** the seal (13) comprises means of reciprocal association with the flange.

10. Sealed container (1) according to claim 4 and one of claims 5 to 9 when they depend on claim 4, **characterized in that** the seal (13) has at least one contact face with at least one of the flange bodies provided with a notching cooperating with a complementary notching provided on the face of said flange body opposite which it is placed, the notching of the contact face of the seal and the complementary notching forming the means of reciprocal association.

11. Sealed container (1) according to claim 4 or one of claims 5 to 10 when they depend on claim 4, **characterized in that** the external flange body (5) comprises an internal peripheral rim (55) abutting which the seal (13) is positioned.

12. Sealed container (1) according to claim 4 or one of claims 5 to 11 when they depend on claim 4, **characterized in that** the external flange body (5) comprises a lower skirt (10) comprising longitudinal slots (11) extending from the lower end edge of the external flange body (5) and evenly distributed over the periphery of said flange body so as to form elastic tabs (12) capable of exerting a clamping force on the internal flange body (6).

13. Sealed container (1) according to claim 4 or one of claims 5 to 12 when they depend on claim 4, **characterized in that** the container body is formed by a flexible film (2) fixed to the flange, the flexible film (2) being held in engagement between the external flange body (5) and the internal flange body (6).

14. Sealed container (1) according to claim 13, **characterized in that** it comprises a clamping ring (20) for clamping the external flange body (5) on the internal flange body (6) arranged to grip the parts of the external flange body (5) and of the internal flange body (6) between which the flexible film is interposed.

15. Sealed container (1) according to claim 13 or claim 14, **characterized in that** it comprises an additional annular seal (8) housed in a peripheral groove provided on the inner face of the external flange body (5), the flexible film (2) extending between the additional annular seal (8) and the internal flange body (6).

16. Sealed container (1) according to one of the preceding claims, **characterized in that** all or part of the container is made of a bacteriostatic material.
